(19) Europäisches Patentamt
European Patent Office
Office européen des brevets

(11) Veröffentlichungsnummer: **0 232 222 B1**

# (12) EUROPÄISCHE PATENTSCHRIFT

(45) Veröffentlichungstag der Patentschrift: **09.09.92**

(51) Int. Cl.[5]: **C07D 487/04**, //(C07D487/04, 209:00,209:00)

(21) Anmeldenummer: **87810050.2**

(22) Anmeldetag: **26.01.87**

Die Akte enthält technische Angaben, die nach dem Eingang der Anmeldung eingereicht wurden und die nicht in dieser Patentschrift enthalten sind.

## (54) Verfahren zur Herstellung von bromierten Pyrrolo-[3,4-c]-pyrrolen.

(30) Priorität: **31.01.86 CH 365/86**

(43) Veröffentlichungstag der Anmeldung:
**12.08.87 Patentblatt 87/33**

(45) Bekanntmachung des Hinweises auf die Patenterteilung:
**09.09.92 Patentblatt 92/37**

(84) Benannte Vertragsstaaten:
**CH DE FR GB IT LI**

(56) Entgegenhaltungen:
**EP-A- 0 061 426**
**EP-A- 0 094 911**
**EP-A- 0 133 156**
**EP-A- 0 184 982**

**Organikum, 1981, 15. Auflage, Kap. 5.1.5, S. 391-395**

**Advanced Organic Chemistry, 1984, 2. Auflage, Kap. 1-12, S. 482-484**

**J. Chem. Soc., 1928, S. 343-347**

**J. Org. Chem., 1975, Band 40, S. 2248-2250**

(73) Patentinhaber: **CIBA-GEIGY AG**
**Klybeckstrasse 141**
**CH-4002 Basel(CH)**

(72) Erfinder: **Wallquist, Olof, Dr.**
**Rte de Confin 31**
**CH-1723 Marly(CH)**
Erfinder: **Iqbal, Abul, Dr.**
**Im Schaiengarten 1**
**CH-4107 Ettingen(CH)**
Erfinder: **Pfenninger, Johannes, Dr.**
**2233 Inwood Road**
**Lancashire, Wilmington, DE 19810(US)**
Erfinder: **Rochat, Alain Claude, Dr.**
**Route de Schiffenen 38**
**CH-1700 Fribourg(CH)**

EP 0 232 222 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von bromierten Pyrrolo-[3,4-c]-pyrrolen durch direkte Bromierung der Pyrrolo-[3,4-c]-pyrrole.

Halogenierte 1,4-Diketo-pyrrolo-[3,4-c]-pyrrole, wie sie z.B. in EP-A 61426, 94 911, 184 982 und 133 156 beschrieben sind, stellen eine wichtige Gruppe von Pigmenten der neuen Klasse der 1,4-Diketo-pyrrolo-[3,4-c]-pyrrole dar. Sie wurden bisher ausgehend von den entsprechenden bromierten Nitrilen durch Umsetzung mit Bromessigester und Zink, gemäss der in EP-A 61426 beschriebenen Methode, oder mit einem Bernsteinsäurediester in Gegenwart einer starken Base, gemäss der in EP-A 94911 beschriebenen Methode, hergestellt. Die für die Herstellung der an den aromatischen Substituenten bromierten 1,4-Diketo-pyrrolo-[3,4-c]-pyrrole benötigten bromierten Nitrile können schlecht direkt aus den aromatischen Nitrilen durch Bromierung erhalten werden, sondern müssen aus einfachen Ausgangsmaterialien in mehrstufigen Prozessen mit niedrigen Ausbeuten und relativ hohen Kosten hergestellt werden. So kann z.B. das Benzonitril nicht einfach und direkt mit Brom in das p-Brombenzonitril übergeführt werden. Letzteres wird durch Bromierung von Toluol (Glinzer & Fittig, Ann. 136, 301 (1865)) und anschliessende Oxidation zum Nitril (Hübner & Wallach, Ann. 154, 293 (1870); DE-OS 1 189 976) hergestellt. Aus D.M. Williams, T.C. James, J.C.S. 1928, 343 und H.J. Reich, I.L. Reich, J. Org. Chem. 40, 2248 (1975) ist bekannt, dass die Bromierung von Verbindungen, die mindestens einen aromatischen Ring und zusätzlich mindestens eine konjugierte aliphatische Doppelbindung enthalten (z.B. Zimtsäurederivate oder 1,4-Diphenyl-1,3-butadien), zu einer Addition an die Doppelbindung anstatt zur Substitution am aromatischen Ring führt.

Bromierte Nitrile, wie sie zur bekannten Herstellung von bromierten 1,4-Diketo-pyrrolo-[3,4-c]-pyrrolen benötigt werden, sind daher schwer und nur über mehrere Stufen zu hohen Preisen zugänglich.

Es ist nun gefunden worden, dass an den aromatischen Substituenten durch Brom substituierte 3,6-Diphenyl-1,4-diketo-pyrrolo[3,4-c]-pyrrole überraschenderweise (insbesondere in Anbetracht der obenerwähnten Lehren von D.M. Williams, T.C. James und H.J. Reich, I.L. Reich) durch direkte Bromierung der entsprechenden 1,4-Diketo-pyrrolo-[3,4-c]-pyrrole, welche leicht aus meist billigen, leicht zugänglichen Nitrilen erhältlich sind, mit hoher Ausbeute hergestellt werden können.

Die vorliegende Erfindung betrifft demnach ein Verfahren zur Herstellung von an den aromatischen Substituenten durch Brom substituierten 3,6-Diphenyl-1,4-diketo-pyrrolo-[3,4-c]-pyrrolen durch Bromierung eines Pyrrolo-[3,4-c]-pyrrols oder eines Gemisches verschiedener Pyrrolo-[3,4-c]-pyrrole der Formel I

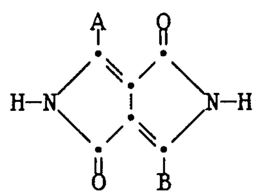

(I),

worin A und B eine Gruppe der Formel

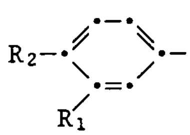

sind, worin $R_1$ und $R_2$ Wasserstoff, $C_1$-$C_4$-Alkyl, $C_1$-$C_4$-Alkoxy oder Phenyl bedeuten, mit einem Bromierungsmittel in einer Menge, die 0,5 bis 70 Aequivalente Brom liefert, mit oder ohne Lösungsmittel, bei Temperaturen zwischen 20 und 150 °C, bis zum gewünschten Bromgehalt und anschliessende Isolierung des bromierten Pyrrolo-[3,4-c]-pyrrols durch Entfernung des überschüssigen Bromierungsmittels und gegebenenfalls des Lösungsmittels nach üblichen Methoden. Bevorzugt geht man von einem einheitlichen Pyrrolo-[3,4-c)-pyrrol aus.

Die Reste A und B sind vorzugsweise gleich.

Beispiele von $C_1$-$C_4$-Alkylgruppen sind Methyl, Ethyl, n-Propyl, Isopropyl, n-Butyl, sec-Butyl, tert.-Butyl.

$C_1$-$C_4$-Alkoxygruppen sind z.B.: Methoxy, Ethoxy, Propoxy, Isopropoxy, n-Butoxy, sec-Butoxy, tert.-Butoxy.

Besonders bevorzugt ist die Bromierung von Pyrrolo-[3,4-c]-pyrrolen der Formel I, worin A und B eine

Gruppe der Formel

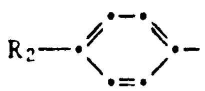

sind, worin $R_2$ Wasserstoff oder Phenyl bedeutet.

Ganz besonders bevorzugt ist die Bromierung von 1,4-Diketopyrrolo-[3,4-c]-pyrrolen der Formel I, worin A und B unsubstituiertes Phenyl bedeuten.

Die zu bromierenden 1,4-Diketopyrrolo-[3,4-c]-pyrrole sind z.B. aus EP-A 61426, EP-A 94911 und EP-A 133156 bekannt und können nach den in den gleichen Publikationen beschriebenen Methoden hergestellt werden.

Das zu bromierende 1,4-Diketopyrrolo-[3,4-c]-pyrrol kann entweder in einem Lösungsmittel oder in einer Schmelze oder in Abwesenheit eines Lösungsmittels mit dem Bromierungsmittel in der gewünschten Menge, d.h. in einer Menge die 0,5 bis 70, bevorzugt 2 bis 40 Aequivalente Brom liefert, bei Temperaturen, die zweckmässig zwischen -20 und 200 °C, bevorzugt zwischen 20 und 150 °C, variieren können, in einem offenen oder geschlossenen Gefäss, bis zum gewünschten Bromgehalt bromiert werden.

Geeignete Bromierungsmittel sind z.B.: Brom, N-Bromsuccinimid, Dibromcyanursäure, Thionylbromid, Alkalimetallhypobromide, N,N-Dibromphenylsulfonamid, Pyridinhydrobromid-perbromid, N-Bromphthalimid. Bevorzugt werden Brom und Dibromcyanursäure.

Als Lösungsmittel können z.B. halogenierte oder nitrierte aromatische Kohlenwasserstoffe, unsubstituierte oder halogenierte aliphatische Kohlenwasserstoffe sowie Mineralsäuren eingesetzt werden. Beispiele dafür sind: o-Dichlorbenzol, Nitrobenzol, Tetrachlorkohlenstoff, 1,1,2,2-Tetrachlorethan, Methylenchlorid, Trichlorethylen, Tetrachlorethylen, Petrolether, Nitroethan, Hexan, Decalin, ®Shellsol TD (ein Gemisch von aliphatischen Kohlenwasserstoffen von Shell), Schwefelsäure.

Um die Bromierung zu beschleunigen, wird die Reaktion mit Vorteil in Gegenwart einer schwachen Base, wie z.B. $K_2CO_3$, durchgeführt. Mit Vorteil können auch oberflächenaktive Mittel, wie z.B. Polyoxyethylen-lauryletherund Sulfobernsteinsäure-bis-2-ethylhexyl-ester-Natriumsalz, sowie Oxidationsmittel, wie z.B. Cer-IV-ammoniumnitrat und Kupfer-II-nitrat, zugegeben werden.

Bevorzugt wird die Bromierung von 1,4-Diketopyrrolo-[3,4-c]-pyrrolen der Formel I mit 2-40 Aequivalenten Brom in einem der obenerwähnten Lösungsmittel oder in einem Gemisch davon, oder in flüssigem oder gasförmigem Brom ohne Lösungsmittel, oder mit Dibromcyanursäure in Schwefelsäure, bei Temperaturen zwischen 20 und 150 °C durchgeführt. Wenn nötig wird das überschüssige Brom durch Zugabe eines geeigneten Reagenz abgefangen und die Suspension abgenutscht. Der Rückstand wird zweckmässig mit einem polaren organischen Lösungsmittel gereinigt und im Vakuumtrockenschrank getrocknet.

Durch die Wahl der Reaktionstemperatur und -dauer und/oder des verwendeten Lösungsmittels und/oder durch Erhöhung bzw. Verminderung der Menge des Bromierungsmittels, kann das erfindungsgemässe Verfahren so gesteuert werden, dass einheitliche Produkte oder Gemische von unbromierten und einheitlich oder unterschiedlich bromierten oder von unterschiedlich bromierten 1,4-Diketopyrrolo-[3,4-c]-pyrrole entstehen.

In der Regel verwendet man die erfindungsgemäss erhaltenen bromierten 1,4-Diketo-pyrrolo-[3,4-c]-pyrrole als Pigmente zum Einfärben von hochmolekularen organischen Materialien. Dabei lassen sich die Pigmente im allgemeinen direkt in der Form einsetzen, wie sie nach dem erfindungsgemässen Verfahren anfallen.

Je nach Art ihrer Substituenten und der zu färbenden Polymeren können die erfindungsgemäss bromierten 1,4-Diketopyrrolo-[3,4-c]-pyrrole der Formel I auch als polymerlösliche Farbstoffe verwendet werden.

Die gemäss der vorliegenden Erfindung hergestellten bromierten 1,4-Diketo-pyrrolo-[3,4-c]-pyrrole zeichnen sich insbesondere durch gute Hitzebeständigkeit aus.

Je nach Verwendungszweck kann es vorteilhaft sein, die erfindungsgemäss erhältlichen Gemische von bromierten und unbromierten oder von verschiedentlich bromierten 1,4-Diketo-pyrrolo-[3,4-c]-pyrrolen einzusetzen.

Von besonderem Interesse sind die ausgehend von Verbindungen der Formel I, worin A und B Phenyl bedeuten, erhaltenen Gemische von bromierten 1,4-Diketo-[3,4-c]-pyrrolen, die einen Bromgehalt von mindestens 10, insbesondere 20 % aufweisen.

Hochmolekulare organische Materialien, die mit den erfindungsgemäss erhaltenen bromierten 1,4-Diketo-pyrrolo-[3,4-c]-pyrrolen der Formel I gefärbt bzw. pigmentiert werden können, sind z.B. Celluloseether und -ester, wie Ethylcellulose, Nitrocellulose, Celluloseacetat oder Cellulosebutyrat, natürliche Harze oder Kunstharze, wie Polymerisationsharze oder Kondensationsharze, z.B. Aminoplaste, insbesondere Harnstoff- und Melamin-Formaldehydharze, Alkydharze, Phenoplaste, Polycarbonate, Polyolefine, wie Polyethylen oder Polypropylen, Polystyrol, Polyvinylchlorid, Polyphenylether, Polyphenylsulfid, Polyacrylnitril, Polyacrylsäureester, Polyamide, Polyurethane oder Polyester, Gummi, Casein, Silikon und Silikonharze, einzeln oder in Mischungen.

Dabei spielt es keine Rolle, ob die erwähnten hochmolekularen organischen Verbindungen als plastische Massen, Schmelzen oder in Form von Spinnlösungen, Lacken, Anstrichstoffen oder Druckfarben vorliegen. Je nach Verwendungszweck erweist es sich als vorteilhaft, die erfindungsgemäss erhaltenen Pigmente als Toner oder in Form von Präparaten einzusetzen. Bezogen auf das zu pigmentierende hochmolekulare organische Material setzt man die Verbindungen der Formel I in einer Menge von vorzugsweise 0,1 bis 10 Gew.% ein.

Die erhaltenen Färbungen, beispielsweise in Kunststoffen, Fasern, Lacken oder Drucken, zeichnen sich durch hohe Farbstärke, gute Dispergierbarkeit, gute Ueberlackier-, Migrations-, Hitze-, Licht- und Wetterbeständigkeit, sowie durch einen guten Glanz und ein gutes IR-Remissionsverhalten aus.

Die erfindungsgemäss bromierten Verbindungen der Formel (1) können auch als photoleitfähige Substanzen, beispielsweise in elektrophotographischen Aufzeichnungsmaterialien Verwendung finden.

Die erfindungsgemäss bromierten Verbindungen der Formel (1) können auch als photoelektrophoretische Toner verwendet werden.

Die erfindungsgemäss bromierten Verbindungen der Formel (I), die in den angewandten Polymeren teilweise oder vollständig gelöst vorliegen, zeichnen sich ebenfalls durch reinen Farbton, hohe Farbstärke, gute allgemeine Echtheiten, insbesondere Licht- und Sublimierechtheit, und ausserdem durch hohe Fluoreszenz aus. Sie eignen sich für die Anwendung in Sonnenenergie-Kollektoren und zur Herstellung von Laser-Strahlen.

Die folgenden Beispiele erläutern die Erfindung.

Beispiel 1: Auf dem Boden eines Exsikkators (Ø = 20 cm) werden eine Kristallisationsschale mit 218 g Brom und auf der Porzellan-Einlage eine mit 50 g 3,6-Diphenyl-1,4-diketopyrrolo-[3,4-c]-pyrrol gefüllte Kristallisationsschale gestellt. Der Deckel wird aufgelegt, der Entlüftungshahn wird leicht geöffnet, und das Ganze wird 8 Tage bei Zimmertemperatur stehen gelassen. Danach wird die Kristallisationsschale mit dem bromierten Produkt entnommen und bis zum konstanten Gewicht stehen gelassen [das Produkt kann zur Entfernung von überschüssigem Brom auch bei 0-5 °C langsam einer Lösung von 1,5 bis 3 Aequivalenten (bezogen auf Brom) Cyclohexen, in 200 ml Tetrachlorkohlenstoff zugegeben und nach 30 Minuten Rühren abgenutscht werden]. Das Rohprodukt wird zur Reinigung in Methanol einige Stunden bei Rückfluss erhitzt, abgenutscht, mit Methanol gewaschen und im Vakuumtrockenschrank bei 70 °C getrocknet. Man erhält 67,0 g (87 % der Theorie) eines roten schwerlöslichen Festkörpers, der in spektroskopischer und koloristischer Hinsicht identisch ist mit 3,6-Di(4-bromphenyl)-1,4-diketopyrrolo-[3,4-c]-pyrrol. Das Produkt ergibt, in PVC eingearbeitet, eine rote Färbung.

| C, H, N, Br-Analyse: | | | | |
|---|---|---|---|---|
| | C | H | H | Br |
| Ber.:<br>Gef.: | 48,46 %<br>49,85 % | 2,26 %<br>2,41 % | 6,28 %<br>6,40 % | 35,8 %<br>33,5 % |

Beispiel 2: In einem 50 ml-Rundkolben mit Rückflusskühler wird ein Gemisch von 1,0 g 3,6-Diphenyl-1,4-diketopyrrolo-[3,4-c]-pyrrol und 3,8 g Brom in 10 ml Tetrachlorkohlenstoff während 18 Stunden bei Zimmertemperatur gerührt. Das Produkt wird abgenutscht, mit viel Tetrachlorkohlenstoff und danach mit Methanol gewaschen und anschliessend während 3 Stunden in 200 ml Methanol bei Rückfluss erhitzt, abgekühlt, mit Methanol gewaschen und im Vakuumtrockenschrank bei 70 °C getrocknet. Man erhält 0,9 g einer Pigmentmischung mit 13,3 % Bromgehalt, die gemäss Feld-Desorption-MS-Analyse aus den Verbindungen der Formeln

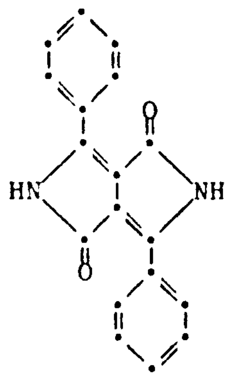
,
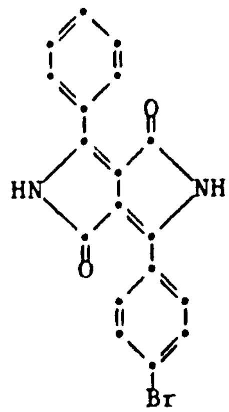
,
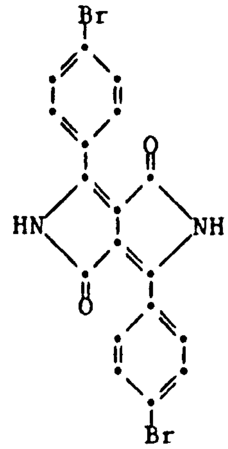

A B C

besteht, und, in PVC eingearbeitet, eine rote Färbung ergibt.

Beispiel 3: In einem 50 ml-Sulfierkolben werden 55,2 g Aluminiumchlorid vorgelegt, und bei 100 °C werden portionenweise 3,4 g NaCl, 2,5 g KCl und 0,9 g NaF zugegeben. Zu der erhaltenen Schmelze werden 2,0 g 3,6-Diphenyl-1,4-diketopyrrolo-[3,4-c]-pyrrol zugegeben und dann innert einer Stunde 2,4 g Brom zugetropft. Das Gemisch wird 4 Stunden bei 100 °C gerührt und anschliessend auf 100 ml 2N HCl/Eis gegossen und abfiltriert. Der Rückstand wird in Methanol während 16 Stunden bei Rückfluss erhitzt, abgekühlt, abgenutscht, mit Methanol gewaschen und im Vakuumtrockenschrank bei 70 °C getrocknet. Man erhält 2,3 g einer Pigmentmischung (bestehend im wesentlichen aus den Komponenten A, B und C) mit einem Bromgehalt von 31,6 %, die, in PVC eingearbeitet, eine rote Färbung ergibt.

Beispiel 4: In einem 100 ml-Rundkolben werden 3,0 g 3,6-Diphenyl-1,4-diketo-pyrrolo-[3,4-c]-pyrrol zu 7,7 ml konzentrierter Schwefelsäure gegeben. Danach wird eine Lösung von 8,7 g Dibromcyanursäure in 70 ml konzentrierter Schwefelsäure langsam zugegeben, und das erhaltene Gemisch wird 15 Minuten bei Zimmertemperatur gerührt. Anschliessend wird das Reaktionsgemisch auf 100 ml Eis gegossen und abgenutscht. Der Rückstand wird in 50 ml verdünnter NaOH-Lösung aufgeschlämmt, abgenutscht, mit Wasser gewaschen und im Vakuumtrockenschrank getrocknet. Der Festkörper wird danach in Methanol während 3 Stunden am Rückfluss erhitzt. Man erhält 2,1 g einer Pigmentmischung (bestehend im wesentlichen aus den Komponenten A, B und C) mit einem Bromgehalt von 43,8 %, die, in PVC eingearbeitet, eine orange Färbung ergibt.

Beispiel 5: In einem 1 1-Emailautoklaven werden in eine mit drei Beinen versehenen Glasschale 5,0 g 3,6-Diphenyl-1,4-diketo-pyrrolo-[3,4-c]-pyrrol gegeben. Auf dem Boden des Autoklaven gibt man 27,8 g Brom, schliesst den Autoklaven ab und heizt auf 100 °C während 18 Stunden. Der Autoklav wird auf Zimmertemperatur abgekühlt, entlüftet, mit Stickstoff gespült, die Glasschale wird entnommen und deren Inhalt zur Entfernung von überschüssigem Brom und zur Vermeidung von Nebenreaktionen bei 0-5 °C langsam einer Lösung von 1,5 bis 3 Aequivalenten (bezogen auf das Brom) eines Olefins, z.B. Cyclohexen, in 200 ml Tetrachlorkohlenstoff zugegeben und 30 Minuten gerührt, dann abgenutscht, zur Reinigung einige Stunden in Methanol am Rückfluss erhitzt, abgenutscht und bei 70 °C im Vakuumtrockenschrank getrocknet. Man erhält 6,5 g eines Festkörpers, der, in PVC eingearbeitet, eine rote Färbung ergibt und in spektroskopischer und koloristischer Hinsicht identisch ist mit 3,6-Di(4-bromphenyl)-1,4-diketopyrrolo-[3,4-c]-pyrrol.

| | | C | H | N | Br |
|---|---|---|---|---|---|
| C, H, N, Br Analyse: | Ber.<br>Gef. | 48,46 %<br>49,78 % | 2,26 %<br>2,44 % | 6,28 %<br>6,33 % | 35,8 %<br>33,8 % |

Beispiel 6: Verfährt man wie im Beispiel 5, führt aber die Reaktion bei 23 °C durch, so erhält man 5,9 g einer Pigmentmischung (bestehend im wesentlichen aus den Komponenten A, B und C) mit einem Bromgehalt von 21,8 %, die, in PVC eingearbeitet, eine rote Färbung ergibt.

Beispiel 7: In einem 1 1-Emailautoklaven werden 30 g 3,6-Diphenyl-1,4-diketopyrrolo-[3,4-c]-pyrrol und 116 g Brom in 300 ml Tetrachlorkohlenstoff während 18 Stunden bei 100 °C gerührt. Nach dem Abkühlen

auf Zimmertemperatur wird die Suspension abgenutscht. Zur Entfernung von überschüssigem Brom gibt man mit Vorteil bei 0-5°C langsam eine Lösung von z.B. 1,5 bis 3 Aequivalenten (bezogen auf Brom) Cyclohexen in 200 ml Tetrachlorkohlenstoff zum abgekühlten Reaktionsgemisch vor dem Abnutschen und rührt ca. 30 Minuten. Das Rohprodukt wird zur Reinigung einige Stunden in Methanol am Rückfluss erhitzt, abgenutscht und im Vakuumtrockenschrank bei 70°C getrocknet. Man erhält 36,9 g einer Pigmentmischung (bestehend im wesentlichen aus den Komponenten A, B und C) mit einem Bromgehalt von 25,8 %, die, in PVC eingearbeitet, eine rote Färbung ergibt.

Beispiel 8: Verfährt man wie in Beispiel 7, verwendet aber anstelle von Tetrachlorkohlenstoff Petrolether 40°-60°C als Lösungsmittel, so erhält man 32,0 g einer Pigmentmischung (bestehend im wesentlichen aus den Komponenten A, B und C) mit einem Bromgehalt von 19,7 %, die, in PVC eingearbeitet, eine rote Färbung ergibt.

Beispiel 9: Verfährt man wie in Beispiel 7, vewendet aber anstelle von Tetrachlorkohlenstoff ®Shellsol TD (Gemisch von aliphatischen Kohlenwasserstoffen der Firma Shell) als Lösungsmittel, so erhält man 34,4 g einer Pigmentmischung (bestehend im wesentlichen aus den Komponenten A, B und C) mit einem Bromgehalt von 12,5 %, die, in PVC eingearbeitet, eine rote Färbung ergibt.

Beispiel 10: Verfährt man wie in Beispiel 7, verwendet aber anstelle von Tetrachlorkohlenstoff o-Dichlorbenzol als Lösungsmittel, so erhält man 35,5 g einer Pigmentmischung (bestehend im wesentlichen aus den Komponenten A, B und C) mit einem Bromgehalt von 26,3 %, die, in PVC eingearbeitet, eine rote Färbung ergibt.

Beispiel 11: Verfährt man wie in Beispiel 7, verwendet aber anstelle von Tetrachlorkohlenstoff Nitrobenzol als Lösungsmittel, so erhält man 26,5 g einer Pigmentmischung (bestehend im wesentlichen aus den Komponenten A, B und C) mit einem Bromgehalt von 31,2 %, die, in PVC eingearbeitet, eine rote Färbung ergibt.

Beispiel 12: Verfährt man wie in Beispiel 7, verwendet aber anstelle von Tetrachlorkohlenstoff 1,1,2,2-Tetrachlorethan als Lösungsmittel, so erhält man 36,5 g einer Pigmentmischung (bestehend im wesentlichen aus den Komponenten A, B und C) mit einem Bromgehalt von 26,7 %, die, in PVC eingearbeitet, eine rote Färbung ergibt.

Beispiel 13: In einem Bombenrohr (100 ml) werden in 30 ml Tetrachlorkohlenstoff, 3,0 g 3,6-Diphenyl-1,4-diketopyrrolo-[3,4-c] pyrrol und 11,4 g Brom während 18 Stunden bei Zimmertemperatur gerührt. Die Aufarbeitung analog Beispiel 2 ergibt 2,8 g einer Pigmentmischung (bestehend im wesentlichen aus den Komponenten A, B und C) mit einem Bromgehalt von 11,1 %, die, in PVC eingearbeitet, eine rote Färbung ergibt.

Beispiel 14: Verfährt man wie in Beispiel 13, gibt aber zusätzlich 0,5 g Sulfobernsteinsäure-bis-2-ethylhexylester-Natriumsalz zu, so erhält man 3,1 g einer Pigmentmischung (bestehend im wesentlichen aus den Komponenten A, B und C) mit einem Bromgehalt von 15 %, die, in PVC eingearbeitet, eine rote Färbung ergibt.

Beispiel 15: Verfährt man wie in Beispiel 13, gibt aber noch 5,7 g $K_2CO_3$ zu, so erhält man 2,9 g einer Pigmentmischung (bestehend im wesentlichen aus den Komponenten A, B und C) mit einem Bromgehalt von 16 %, die, in PVC eingearbeitet, eine rote Färbung ergibt.

Beispiel 16: Verfährt man wie in Beispiel 1, verwendet aber als Ausgangsmaterial 3,0 g 3,6-Di-(4-phenyl-phenyl)-1,4-diketopyrrolo-[3,4-c]-pyrrol und 7,3 g Brom, so erhält man 3,7 g einer Pigmentmischung mit einem Bromgehalt von 35,7 %, die, in PVC eingearbeitet, eine rotviolette Färbung ergibt.

Beispiel 17: Zu 30 ml Brom werden 3,0 g 3,6-Diphenyl-1,4-diketopyrrolo-[3,4-c]-pyrrol gegeben und eine Stunde bei Zimmertemperatur gerührt. Das Reaktionsgemisch wird abgenutscht, mit Petrolether gewaschen, und der Rückstand wird während drei Stunden in Methanol bei Rückfluss erhitzt, abgenutscht, mit Methanol gewaschen und im Vakuumschrank bei 70°C getrocknet. Man erhält 3,2 g einer Pigmentmischung (bestehend im wesentlichen aus den Komponenten A, B und C) mit einem Bromgehalt von 21,9 % Br, die, in PVC eingearbeitet, eine rote Färbung ergibt.

Beispiel 18: Verfährt man wie in Beispiel 1, verwendet aber anstelle von 50 g 3,6-Diphenyl-1,4-diketopyrrolo-[3,4-c]-pyrrol und 278 g Brom 0,5 g 3,6-Di-(3-methoxyphenyl)-1,4-diketopyrrolo-[3,4-c]-pyrrol und 7,3 g Brom, so erhält man 0,5 g einer Pigmentmischung mit 48,8 % Bromgehalt, die, in PVC eingearbeitet, eine rotviolette Färbung ergibt.

Beispiel 19: Verfährt man wie in Beispiel 7, verwendet aber 232 g Brom, so erhält man 40,3 g einer Pigmentmischung (bestehend im wesentlichen aus den Komponenten A, B und C) mit einem Bromgehalt von 32,4 %, die, in PVC eingearbeitet eine rote Färbung ergibt.

Beispiel 20: Verfährt man wie in Beispiel 7, verwendet aber anstelle von Tetrachlorkohlenstoff o-Dichlorbenzol und 232 g Brom, so erhält man 36,7 g einer Pigmentmischung (bestehend im wesentlichen aus den Komponenten A, B und C) mit einem Bromgehalt von 30,6 %, die in PVC eingearbeitet eine rote

Färbung ergibt.

**Patentansprüche**

1. Verfahren zur Herstellung von an den aromatischen Substituenten durch Brom substituierten 3,6-Diphenyl-1,4-diketo-pyrrolo-[3,4-c]-pyrrolen durch Bromierung eines Pyrrolo-[3,4-c]-pyrrols oder eines Gemisches verschiedener Pyrrolo-[3,4-c]-pyrrole der Formel I

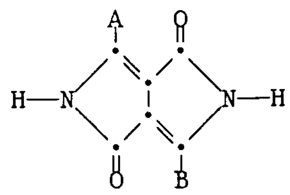

(I),

worin A und B gleiche oder verschiedene Reste der Formel

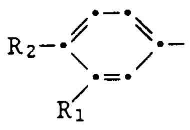

sind, worin $R_1$ und $R_2$ Wasserstoff, $C_1$-$C_4$-Alkyl, $C_1$-$C_4$-Alkoxy oder Phenyl bedeuten, mit einem Bromierungsmittel in einer Menge, die 0,5 bis 70 Aequivalente Brom liefert, mit oder ohne Lösungsmittel, bei Temperaturen zwischen 20 und 150° C, bis zum gewünschten Bromgehalt und anschliessende Isolierung des bromierten Pyrrolo-[3,4-c]-pyrrols durch Entfernung des überschüssigen Bromierungsmittels und gegebenenfalls des Lösungsmittels nach üblichen Methoden.

2. Verfahren gemäss Anspruch 1, dadurch gekennzeichnet, dass man von einem einheitlichen Pyrrolo-[3,4-c]-pyrrol der Formel I ausgeht.

3. Verfahren gemäss Anspruch 1, dadurch gekennzeichnet, dass man von einem Pyrrolo-[3,4-c]-pyrrol der Formel I ausgeht, worin A und B gleich sind.

4. Verfahren gemäss Anspruch 1, dadurch gekennzeichnet, dass man von einem Pyrrolo-[3,4-c]-pyrrol der Formel I ausgeht, worin A und B eine Gruppe der Formel

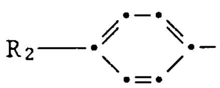

sind, worin $R_2$ Wasserstoff oder Phenyl bedeutet.

5. Verfahren gemäss Anspruch 1, dadurch gekennzeichnet, dass man von einem Pyrrolo-[3,4-c]-pyrrol der Formel I ausgeht, worin A und B unsubstituiertes Phenyl bedeuten.

6. Verfahren gemäss Anspruch 1, dadurch gekennzeichnet, dass man als Bromierungsmittel Brom oder Dibromcyanursäure einsetzt.

7. Verfahren gemäss Anspruch 1, dadurch gekennzeichnet, dass man als Bromierungsmittel Brom in einem Lösungsmittel verwendet.

8. Verfahren gemäss Anspruch 1, dadurch gekennzeichnet, dass man als Bromierungsmittel Brom ohne Lösungsmittel einsetzt.

## Claims

**1.** Process for the preparation of 3,6-diphenyl-1,4-diketo-pyrrolo-[3,4-c]-pyrroles substituted by bromine on the aromatic substituents by bromination of a pyrrolo-[3,4-c]-pyrrole or a mixture of different pyrrolo-[3,4-c]-pyrroles of the formula I

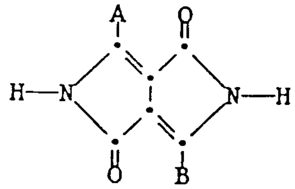

(I),

in which A and B are identical or different radicals of the formula

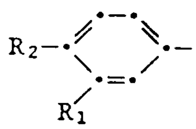

in which $R_1$ and $R_2$ are hydrogen, $C_1$-$C_4$alkyl, $C_1$-$C_4$alkoxy or phenyl, with a brominating agent in an amount which supplies 0.5 to 70 equivalents of bromine, with or without a solvent, at temperatures between 20 and 150 ° C, until the desired bromine content is obtained, and subsequent isolation of the brominated pyrrolo-[3,4-c]-pyrrole by removal of the excess brominating agent and, if necessary, of the solvent by customary methods.

**2.** Process according to claim 1, characterized in that a single pyrrolo-[3,4-c]-pyrrole of the formula I is used as the starting substance.

**3.** Process according to claim 1, characterized in that a pyrrolo-[3,4-c]pyrrole of the formula I in which A and B are identical is used as the starting substance.

**4.** Process according to claim 1, characterized in that a pyrrolo-[3,4-c]-pyrrole of the formula I in which A and B are a group of the formula

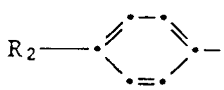

,

in which $R_2$ is hydrogen or phenyl, is used as the starting substance.

**5.** Process according to claim 1, characterized in that a pyrrolo-[3,4-c]-pyrrole of the formula I in which A and B are unsubstituted phenyl is used as the starting substance.

**6.** Process according to claim 1, characterized in that bromine or dibromocyanuric acid is used as the brominating agent.

**7.** Process according to claim 1, characterized in that bromine in a solvent is used as the brominating agent.

**8.** Process according to claim 1, characterized in that bromine without a solvent is used as the brominating agent.

## Revendications

1. Procédé de préparation de 3,6-diphényl-1,4-dicéto-pyrrolo-[3,4-c]-pyrroles bromés sur les substituants aromatiques par bromation d'un pyrrolo-[3,4-c]-pyrrole ou de plusieurs de ces pyrroles à la fois, de formule I :

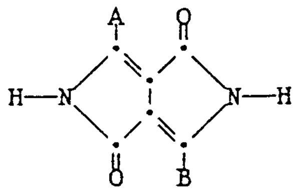

(I)

(dans laquelle A et B sont des groupes identiques ou différents de formule :

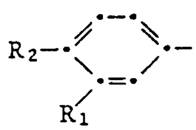

$R_1$ et $R_2$ désignant l'hydrogène, un alkyle ou un alcoxy en $C_1$-$C_4$ ou un phényle), avec un agent de bromation dans une proportion donnant de 0,5 à 70 équivalents de brome, avec ou sans solvant, à des températures de 20 à 150 ° C, jusqu'à la teneur voulue en brome, puis on isole le pyrrolo-[3,4-c]-pyrrole bromé formé en éliminant par des méthodes courantes l'excès de l'agent bromant et le cas échéant le solvant.

2. Procédé selon la revendication 1, caractérisé en ce que l'on part d'un pyrrolo-[3,4-c]-pyrrole de formule I unique, c'est-à-dire de structure chimique bien définie.

3. Procédé selon la revendication 1, caractérisé en ce que l'on part d'un pyrrolo-[3,4-c]-pyrrole de formule I dont les radicaux A et B sont identiques.

4. Procédé selon la revendication 1, caractérisé en ce que l'on part d'un pyrrolo-[3,4-c]-pyrrole de formule I dont les radicaux A et B sont un groupe de formule :

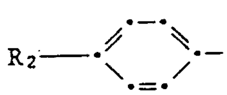

$R_2$ désignant l'hydrogène ou le groupe phényle.

5. Procédé selon la revendication 1, caractérisé en ce que l'on part d'un pyrrolo-[3,4-c]-pyrrole de formule I dont les radicaux A et B sont des phényles sans substituants.

6. Procédé selon la revendication 1, caractérisé en ce que l'on emploie comme agent de bromation du brome ou de l'acide dibromocyanurique.

7. Procédé selon la revendication 1, caractérisé en ce que l'on emploie comme agent de bromation du brome dans un solvant.

8. Procédé selon la revendication 1, caractérisé en ce que l'on emploie comme agent de bromation du brome sans solvant.